(19) Europäisches Patentamt
European Patent Office
Office européen des brevets

(11) EP 2 747 159 A1

(12) **EUROPEAN PATENT APPLICATION**
published in accordance with Art. 153(4) EPC

(43) Date of publication:
**25.06.2014 Bulletin 2014/26**

(21) Application number: **12824426.6**

(22) Date of filing: **14.08.2012**

(51) Int Cl.:
***H01L 51/42*** *(2006.01)* ***C07C 49/92*** *(2006.01)*

(86) International application number:
**PCT/JP2012/005147**

(87) International publication number:
**WO 2013/024595 (21.02.2013 Gazette 2013/08)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.08.2011 JP 2011178394**
**14.10.2011 JP 2011227430**

(71) Applicant: **JX Nippon Oil & Energy Corporation**
**Chiyoda-ku**
**Tokyo 100-8162 (JP)**

(72) Inventors:
- **ICHIBAYASHI, Taku**
  **Tokyo 100-8162 (JP)**
- **ASANO, Tsuyoshi**
  **Tokyo 100-8162 (JP)**

(74) Representative: **Algemeen Octrooi- en Merkenbureau B.V.**
**P.O. Box 645**
**5600 AP Eindhoven (NL)**

# (54) PHOTOELECTRIC CONVERSION ELEMENT AND METHOD FOR MANUFACTURING SAME

(57) A photoelectric conversion element 10 has a structure in which an electron transport layer 40, a photoelectric conversion layer 50, and a hole transport layer 60 are sandwiched between a first electrode 30 and a second electrode 70. The electron transport layer 40 contains a substance represented by the following chemical formula and a reactant thereof:

$$M(X)a \qquad (1)$$

wherein M is selected from the group consisting of alkali metals, alkaline earth metals, group 2B and 3B metals, and transition metals; X is selected from a halogen, a carboxylate group, an alkoxy group, an alkyl group, and an acetonate group represented by the following formula; and a is a positive integer determined in accordance with the valence of M:

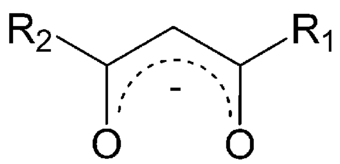

wherein $R_1$ and $R_2$ are selected from hydrogen, a $C_{1-20}$ linear or branched alkyl group, and a $C_{1-20}$ linear or branched alkoxy group, and $R_1$ and $R_2$ may or may not be the same as each other.

FIG. 1

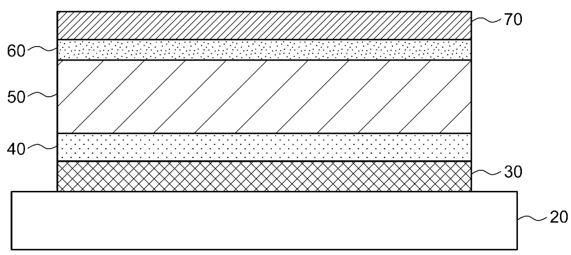


10

EP 2 747 159 A1

**Description**

TECHNICAL FIELD

The present invention relates to a photoelectric conversion element configured to convert light energy into electric energy by photoelectric conversion.

BACKGROUND ART

Because an organic solar cell (photoelectric conversion element) is rich in flexibility; its area and weight can be expected to be enlarged and reduced, respectively; and a simple and inexpensive manufacturing method can be expected, it is considered to be a promising next generation solar cell. A large improvement in a conversion efficiency is currently an important issue towards practical use of organic solar cells.

Various acceptor materials are examined for the improvement in the photoelectric conversion efficiency of photoelectric conversion elements (organic solar cells). In conventional photoelectric conversion elements, open circuit voltages are intended to be increased by making the LUMO (the lowest unoccupied molecular orbital) levels of acceptor materials to be lower than that of PCBM. The LUMO levels of these acceptor materials are different from that of PCBM, and hence the materials for electron transport layers that are suitable for the acceptor materials are required to have characteristics different from the previous ones.

On the other hand, electron transport layers for efficiently transporting the electrons generated in photoelectric conversion layers are under development. Until now, $TiO_x$, $Cs_2CO_3$, and ZnO, etc., are known as electron transport layers.

[Related Art Documents]

[Patent Documents]

[Non-Patent Document 1] A. K. K .Kyaw, X. W. Sun, C. Y. Jiang, G. Q. Lo, D. W. Zhao, D. L. Kwong, Applied Physics Letters 93, 221107 (2008)

[Non-Patent Document 2] P. de Bruyn, D. J. D. Moet, P. W. M. Blom, Organic Electronics, 11, 1419-1422 (2010)

[Non-Patent Document 3] C. Waldauf, M. Morana, P. Denk, P. Schilinsky, K. Coakley, S. A. Choulis, C. J. Brabec, Applied Physics Letters, 89, 233517 (2006)

[Non-Patent Document 4] Hua-Hsien Liao, Li-Min Chen, Zheng Xu, Gang Li, and Yang Yang, APPLIED PHYSICS LETTERS 92, 173303 (2008)

DISCLOSURE OF THE INVENTION

Problem to be Solved by the Invention

When $TiO_x$ is used in an electron transport layer, a manufacturing process of a solution that becomes a precursor of $TiO_x$ is complicated, and further it is needed to leave the solution at rest under the atmosphere for a long period of time for the reaction from the precursor to $TiO_x$, and hence a decrease in productivity cannot be avoided.

When $Cs_2CO_3$ is used in an electron transport layer, a solar cell performance is not exhibited, unless the thickness of the electron transport layer is drastically reduced to a level of several nanometers, because the conductivity of $Cs_2CO_3$ is low. However, it is difficult in a conventional film-forming method, such as a spin coating method, die coating method, or the like, to form an electron transport layer such that the thickness thereof is uniformly at a level of several nanometers, and hence, it is difficult to make the size of the element to be large. Further, if the thickness of the electron transport layer is uneven, it becomes difficult to surely prevent a contact between a photoelectric conversion layer and an electron extraction electrode. If the photoelectric conversion layer and the electron extraction electrode contact each other, a conversion efficiency is drastically decreased, and hence a yield, at which elements having a high conversion efficiency are obtained, is decreased.

When ZnO is used in an electron transport layer, high-temperature firing (300°C) is required, and hence a plastic material having low heat resistance cannot be used as a substrate.

The present invention has been made in view of these problems, and a purpose of the invention is to provide a technique in which: an improvement in the photoelectric conversion efficiency of a photoelectric conversion element can be achieved; the size of the element can be made large; and a plastic substrate can be used.

Means for Solving the Problem

An aspect of the present invention is a photoelectric conversion element. The photoelectric conversion element comprises: a photoelectric conversion layer; an electron extraction electrode provided on one major surface side of the photoelectric conversion layer; a hole extraction electrode provided on the other major surface side of the photoelectric conversion layer; and an electron transport layer provided between the photoelectric conversion layer and the electron extraction electrode, in which the electron transport layer contains a substance represented by the following chemical formula and a reactant thereof:

$$M(X)a \qquad (1)$$

wherein M is selected from the group consisting of alkali metals, alkaline earth metals, group 2B and 3B metals, and transition metals; X is selected from a halogen, a carboxylate group, an alkoxy group, an alkyl group, and an acetonate group represented by the following formula; and a is a positive integer determined in accordance with the valence of M:

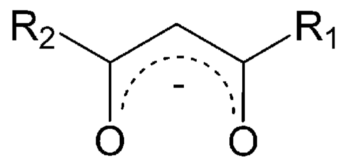

wherein $R_1$ and $R_2$ are selected from hydrogen, a $C_{1-20}$ linear or branched alkyl group, and a $C_{1-20}$ linear or branched alkoxy group, and $R_1$ and $R_2$ may or may not be the same as each other.

According to the photoelectric conversion element of the aforementioned aspect, a photoelectric conversion efficiency can be improved. Further, because the electron transport layer of the aspect can be formed at a relatively low temperature, a photoelectric conversion element can be formed on a plastic substrate having low heat resistance. Further, because the conductivity of the electron transport layer is higher than that of $Cs_2CO_3$, the electron transport layer can be formed so as to have a thickness of approximately 20 to 60 nm, and hence occurrence of a short circuit can be suppressed, even if the thickness of the electron transport layer is a little uneven. Thus, because the electron transport layer can be formed so as to have a large thickness, it can be formed by using various film-forming methods, such as a spin coating method and a die coating method, thereby allowing the size of the photoelectric conversion element to be made large.

In the photoelectric conversion element of the aforementioned aspect, X in the chemical formula (1) may be a carboxylate group or an acetonate group, and the carboxyl group absorption coefficient ($\alpha_1$) in the electron transport layer may be $0.5 \times 10^5$ cm$^{-1} \leq \alpha_1 \leq 2.5 \times 10^5$ cm$^{-1}$. The ionization potential of the electron transport layer may be 6.2 eV or less. The electron transport layer may contain one or more metal compounds and a reactant thereof, the metal compounds being selected from the group consisting of zinc acetate, magnesium acetate, aluminum acetylacetonate, aluminum chloride, gallium acetylacetonate, gallium chloride, zinc acetylacetonate, zinc chloride, and diethylzinc. The photoelectric conversion layer may contain a fullerene derivative having a first reduction potential of 1160 mV (vs $Fc/Fc^+$) or more. The fullerene derivative may be ICBA (bisindenyl C60).

Another aspect of the present invention is a method of manufacturing a photoelectric conversion element. The method is a method of manufacturing a photoelectric conversion element including: a pair of electrodes; a photoelectric conversion layer located between the pair of electrodes; and an electron transport layer provided between one of the electrodes and the photoelectric conversion layer, and the method comprises forming the electron transport layer by heating a film at a temperature ($t_1$) of 100°C $\leq t_1 \leq$ 150°C after the film is formed by coating a solution containing a metal carboxylate.

An aspect in which the respective components described above are appropriately combined can be encompassed within the scope of the invention for which protection is sought by this application.

Advantage of the Invention

According to the present invention, the photoelectric conversion efficiency of a photoelectric conversion element can be improved; the size of the element can be made large; and a plastic substrate can be used.

BRIEF DESCRIPTION OF THE DRAWING

Fig. 1 is a schematic sectional view illustrating a structure of a photoelectric conversion element according to an embodiment.

MODE FOR CARRYING OUT THE INVENTION

Hereinafter, embodiments of the present invention will be described with reference to the accompanying drawing. Fig. 1 is a schematic sectional view illustrating a structure of a photoelectric conversion element 10 according to an embodiment. The photoelectric conversion element 10 of the present embodiment is an organic thin-film solar cell that includes a photoelectric conversion layer containing an organic semiconductor.

The photoelectric conversion element 10 according to the present embodiment comprises a substrate 20, a first electrode 30, an electron transport layer 40, a photoelectric conversion layer 50, a hole transport layer 60, and a second electrode 70.

In the present embodiment, the first electrode 30 is a negative electrode (electron extraction electrode) and is electrically connected to the later-described photoelectric conversion layer 50 via the electron transport layer 40. The first electrode 30 is located on a light-receiving surface side of the photoelectric conversion layer 50 and is formed of: a conductive metal oxide, such as ITO (Indium Tin Oxide), $SnO_2$, FTO (Fluorine doped Tin Oxide), ZnO, AZO (Aluminum doped Zinc Oxide), IZO (Indium doped Zinc Oxide), or the like; a metal thin film, such as gold, silver, copper, aluminum, or the like; or a transparent conducting film, such as a mesh, a stripe, or the like. The first electrode 30 is formed on the light-transmissive substrate 20 so as not to inhibit a light-receiving performance. The substrate 20 may be formed, for example, of colorless or colored glass, wire glass, a glass block, or the like; or a colorless or colored transparent resin. Specific examples of such a resin include polyester, such as polyethylene terephthalate, polyamide, polysulfone, polyether sulfone, polyether ether ketone, polyphenylene sulfide, polycarbonate, polyimide, polymethylmethacrylate, polystyrene, tri-cellulose acetate, and polymethyl pentene, etc.

The electron transport layer 40 is provided in a region between the first electrode 30 and the photoelectric conversion layer 50. The electron transport layer 40 functions to facilitate the transport of electrons from the photoelectric conversion layer 50 to the first electrode 30. The electron transport layer 40 can also function such that the transport of holes from the photoelectric conversion layer 50 to the first electrode 30 is hardly caused. The thickness of the electron transport layer 40 is not particularly limited, but it is, for example, 10 to 100 nm, and preferably 20 to 60 nm.

The electron transport layer 40 contains a substance represented by the following chemical formula and a reactant thereof:

M(X)a (1)

wherein M is selected from the group consisting of alkali metals, alkaline earth metals, group 2B and 3B metals, and transition metals; X is selected from a halogen, a carboxylate group, an alkoxy group, an alkyl group, and an acetonate group represented by the following formula; and a is a positive integer determined in accordance with the valence of M:

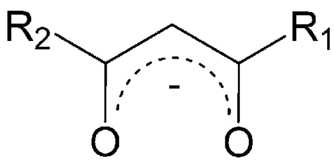

wherein $R_1$ and $R_2$ are selected from hydrogen, a $C_{1-20}$ linear or branched alkyl group, and a $C_{1-20}$ linear or branched alkoxy group, and $R_1$ and $R_2$ may or may not be the same as each other. Examples of the alkyl group include, for example, a methyl group, ethyl group, and propyl group, etc.; and examples of the alkoxy group include, for example, a methoxy group and ethoxy group, etc.

Specific examples of M include Li, Na, Mg, Al, K, Ca, Sc, Ti, V, Cr, Mn, Fe, Co, Ni, Cu, Zn, Ga, Rb, Sr, Zr, Mo, Ru, Rh, Pd, Ag, Cd, In, Sn, Cs, Ba, La, Ir, Pt, Hg, Tl, Pb, ans Bi, etc. Specific examples of X include: ions of halogens, such as fluorine, chlorine, bromine, and iodine, etc.; carboxylate groups derived from carboxylic acids, such as formic acid, acetic acid, propionic acid, butyric acid, oxalic acid, malonic acid, succinic acid, glutaric acid, phthalic acid, acrylic acid, methacrylic acid, citric acid, ethylenediaminetetraacetate, and benzoic acid, etc. The alkoxy group to be used in X is not particularly limited, but $C_{1-10}$ linear or branched alkoxy groups, such as, for example, a methoxy group, ethoxy group, and propoxy group, can be cited. The alkyl group to be used in X is not particularly limited, but $C_{1-10}$ linear or branched alkyl groups, such as a methyl group, ethyl group, and propyl group, can be cited.

More specific examples of the substance to be contained in the electron transport layer 40 include lithium fluoride, lithium chloride, lithium iodide, magnesium chloride, zinc chloride, aluminum chloride, gallium chloride, nickel chloride, gallium chloride, lithium formate, sodium formate, magnesium formate, potassium formate, calcium formate, manganese formate, nickel formate, copper formate, zinc formate, rubidium formate, strontium formate, cesium formate, barium, formate, thallium formate, lead formate, lithium acetate, sodium acetate, magnesium acetate, aluminum acetate, potassium acetate, calcium acetate, chromium acetate, manganese acetate, iron acetate, cobalt acetate, nickel acetate,

copper acetate, zinc acetate, rubidium acetate, strontium acetate, zirconium acetate, molybdenum acetate, rhodium acetate, palladium acetate, silver acetate, cadmium acetate, indium acetate, tin acetate, cesium acetate, barium acetate, mercury acetate, thallium acetate, lead acetate, bismuth acetate, lithium propionate, sodium propionate, magnesium propionate, potassium propionate, calcium propionate, manganese propionate, nickel propionate, zinc propionate, strontium propionate, palladium propionate, barium propionate, lead propionate, lithium butyrate, sodium butyrate, magnesium butyrate, potassium butyrate, calcium butyrate, manganese butyrate, nickel butyrate, zinc butyrate, strontium butyrate, barium butyrate, lead butyrate, aluminum acetylacetonate, scandium acetylacetonate, vanadium acetylacetonate, chromium acetylacetonate, manganese acetylacetonate, iron acetylacetonate, cobalt acetylacetonate, nickel acetylacetonate, copper acetylacetonate, zinc acetylacetonate, gallium acetylacetonate, zirconium acetylacetonate, ruthenium acetylacetonate, rhodium acetylacetonate, palladium acetylacetonate, silver acetylacetonate, indium acetylacetonate, lanthanum acetylacetonate, neodymium acetylacetonate, samarium acetylacetonate, europium acetylacetonate, gadolinium acetylacetonate, terbium acetylacetonate, erbium acetylacetonate, iridium acetylacetonate, platinum acetylacetonate, thallium acetylacetonate, lead acetylacetonate, sodium oxalate, sodium hydrogen oxalate, potassium oxalate, potassium hydrogen oxalate, lithium oxalate, lithium hydrogen oxalate, calcium oxalate, barium oxalate, magnesium oxalate, zinc oxalate, manganese oxalate, nickel oxalate, strontium oxalate, lead oxalate, ammonium malonate, ammonium hydrogen malonate, sodium malonate, sodium hydrogen malonate, potassium malonate, potassium hydrogen malonate, lithium malonate, lithium hydrogen malonate, calcium malonate, barium malonate, magnesium malonate, zinc malonate, manganese malonate, nickel malonate, strontium malonate, lead malonate, ammonium succinate, sodium succinate, potassium succinate, lithium succinate, calcium succinate, barium succinate, magnesium succinate, zinc succinate, manganese succinate, nickel succinate, strontium succinate, lead succinate, ammonium glutarate, sodium glutarate, potassium glutarate, lithium glutarate, calcium glutarate, barium glutarate, magnesium glutarate, zinc glutarate, manganese glutarate, nickel glutarate, strontium glutarate, lead glutarate, ammonium phthalate, sodium phthalate, potassium phthalate, lithium phthalate, calcium phthalate, barium phthalate, magnesium phthalate, zinc phthalate, nickel phthalate, strontium phthalate, and lead phthalate, etc. Among them, zinc acetate, magnesium acetate, aluminum acetylacetonate, aluminum chloride, gallium acetylacetonate, gallium chloride, zinc acetylacetonate, zinc chloride, and diethylzinc are preferred as the substance to be contained in the electron transport layer 40. The reactants of the aforementioned substances refer to intermediate products that have been partially or wholly hydrolyzed or that have been partially condensed. Specifically, for example, a reactant, which is formed by coating a solution containing the aforementioned substance onto a substrate and then by heating the solution at a temperature ($t_1$) of $100°C \leq t_1 \leq 150°C$, is preferred; or when X in the chemical formula (1) is a carboxylate group or an acetonate group, a reactant, which is contained in an electron transport layer having a carboxyl group absorption coefficient ($\alpha_2$) of $0.5 \times 10^5\ cm^{-1} \leq \alpha_2 \leq 2.5 \times 10^5\ cm^{-1}$, is preferred.

The ionization potential of the electron transport layer 40 is preferably 6.2 eV or less, more preferably 6.0 eV or less, and still more preferably 5.8 eV or less. The ionization potential of the electron transport layer 40 can be measured by using photoelectron spectroscopy, as described later.

The electron transport layer 40 of the present embodiment can be formed by coating a solution containing a material represented by the aforementioned chemical formula (1) and then by heating the solution at a relatively low temperature ($t_1$) of $100°C \leq t_1 \leq 150°C$. If the heating temperature is lower than 100°C, the film does not function as an electron transport layer, and hence the photoelectric conversion performance is drastically decreased, or photoelectric conversion is not performed at all. On the other hand, if the heating temperature is higher than_150°C, the ionization potential becomes too high, and hence the photoelectric conversion performance is decreased. The aforementioned solution can be manufactured by dissolving the material represented by the chemical formula (1) in a predetermined solvent. The solvent is not particularly limited, as far as the material represented by the chemical formula (1) can be dissolved therein, but examples thereof include alcohol-based solvents, such as methanol, ethanol, isopropanol, 1-propanol, 2-methoxyethanol, and 2-ethoxyethanol, etc., and mixtures thereof. The concentration of the material represented by the chemical formula (1) in the solution is not particularly limited, but it is 1 mg to 1 g/ml, preferably 5 mg to 500 mg/ml, and more preferably 10 mg to 100 mg /ml. Examples of the material suitable for forming the electron transport layer include zinc acetate, magnesium acetate, aluminum acetylacetonate, aluminum chloride, gallium acetylacetonate, gallium chloride, zinc acetylacetonate, zinc chloride, and diethylzinc, and among them, zinc acetate is most preferred.

The photoelectric conversion layer 50 of the present embodiment is a bulk heterojunction layer, and is formed by mixing, at a nano level, a p-type organic semiconductor having an electron donating property and an n-type organic semiconductor having an electronic accepting property. Examples of the p-type organic semiconductor include electron donating molecules, such as charge transfer agents and charge transfer complexes, the charge transfer agents including: polythiophenes, such as poly (3-hexylthiophene), and oligomers thereof; organic pigment molecules, such as polypyrrole, polyaniline, polyfuran, polypyridine, polycarbazole, phthalocyanine, porphyrin, and perylene, and derivatives and transition metal complexes thereof; triphenylamine compounds; and hydrazine compounds, and the charge transfer complexes including tetra rear full Burren (TTF), etc.

Examples of the n-type organic semiconductor include: fullerene and fullerene derivatives, such as [60]PCBM

(phenyl C61 butyric acid methyl ester), bis[60]PCBM, ICMA (monoindenyl C60), ICBA (bisindenyl C60), and [70]PCBM (phenyl C71 butyric acid methyl ester); carbon materials, such as carbon nanotube and chemically-modified carbon nanotube; and metal complexes having, as a legand, condensed ring aromatic compounds (naphthalene derivatives, anthracene derivatives, phenanthrene derivatives, tetracene derivatives, pyrene derivatives, perylene derivatives, and fluoranthene derivatives), heterocyclic compounds having 5 to 7 members that contain a nitrogen atom, an oxygen atom, and a sulfur atom (e.g., pyridine, pyrazine, pyrimidine, pyridazine, triazine, quinoline, quinoxaline, quinazoline, phthalazine, cinnoline, isoquinoline, pteridine, acridine, phenazine, phenanthroline, tetrazole, pyrazole, imidazole, thiazole, oxazole, indazole, benzimidazole benzotriazol, benzoxazole, benzothiazole, carbazole, purine, triazolopyridazine, triazolopyrimidine, tetrazaindene, oxadiazole, imidazopyridine, pyrralidine, pyrrolopyridine, thiadiazolopyridine, dibenzazepine, and tribenzazepine, etc.), polyarylene compounds, fluorene compounds, cyclopentadiene compounds, silyl compounds, or nitrogen-containing heterocyclic compounds. Among them, fullerene and fullerene derivatives are preferred. Herein, the fullerene represents C60, C70, C76, C78, C80, C82, C84, C90, C96, C240, C540, mixed fullerene, and fullerene nanotube, and the fullerene derivatives represent compounds in which a substituent group is added to the fullerenes.

In the present description, when a specific portion is referred to as a "group", it means that the portion itself may or may not be substituted by one or more (up to the possible largest number) substituent groups. For example, an "alkyl group" means a substituted or unsubstituted alkyl group. The substituent group that can be used in a compound in the present description may be any substituent group.

When such a substituent group is represented by W, the substituent group represented by W is not particularly limited, and may be any substituent group. Examples of the substituent group represented by W include, for example: halogen atoms; alkyl groups (including a cycloalkyl group, bicycloalkyl group, and tricycloalkyl group); alkenyl groups (including a cycloalkenyl group and bicycloalkenyl group); alkynyl groups; aryl groups; heterocyclic groups (also referred to as hetero ring groups); cyano groups; hydroxyl groups; nitro groups; carboxyl groups; alkoxy groups; aryloxy groups; silyloxy groups; heterocyclic oxy groups; acyloxy groups; carbamoyloxy groups; alkoxy carbonyloxy groups; aryloxy carbonyloxy groups; amino groups (including an anilino group); ammonio groups; acylamino groups; aminocarbonyl amino groups; alkoxycarbonylamino groups; aryloxycarbonylamino groups; sulfamoylamino groups; alkyl or aryl sulfonylamino groups; mercapt groups; alkylthio groups; arylthio groups; heterocyclic thio groups; sulfamoyl groups; sulfo groups; alkyl or aryl sulfinyl groups; alkyl or aryl sulfonyl groups; acyl groups; aryloxy carbonyl groups; alkoxycarbonyl groups; carbamoyl groups; aryl or heterocyclic azo groups; imido groups; phosphino groups; phosphinyl groups; phosphinyloxy groups; phosphinyl amino groups; phosphono groups; silyl groups; hydrazino groups; ureido groups; boronic acid groups ($-B(OH)_2$); phosphato groups ($-OPO(OH)_2$); sulfato groups ($-OSO_3H$); and other publicly-known substituent groups.

In more detail, W represents: halogen atoms (e.g., a fluorine atom, chlorine atom, bromine atom, and iodine atom); and alkyl groups [linear, branched, and cyclic substituted or unsubstituted alkyl groups]. Examples of them include: alkyl groups (preferably $C_{1-30}$ alkyl groups, such as, for example, methyl, ethyl, n-propyl, isopropyl, t-butyl, n-octyl, eicosyl, 2-chloroethyl, 2-cyanoethyl, 2-ethylhexyl); cycloalkyl groups (preferably $C_{3-30}$ substituted or unsubstituted cycloalkyl groups, such as, for example, a cyclohexyl group, cyclopentyl group, and 4-n-dodecylcyclohexyl group); bicycloalkyl groups (preferably $C_{5-30}$ substituted or unsubstituted bicycloalkyl groups, i.e., monovalent groups obtained by removing one hydrogen atom from $C_{5-30}$ bicycloalkanes, such as, for example, a bicyclo[1,2,2]heptane-2-yl group and bicyclo[2,2,2]octane-3-yl group); and tricyclo structures having more cyclic structures, etc. The alkyl groups in the substituent groups described below (e.g., an alkyl group in an alkylthio group) represent alkyl groups of such a concept, and are meant to further include alkenyl groups and alkynyl groups.]; and alkenyl groups [linear, branched, and cyclic substituted or unsubstituted alkenyl groups]. Examples of them include: alkenyl groups (preferably $C_{2-30}$ substituted or unsubstituted alkenyl groups, such as, for example, a vinyl group, allyl group, prenyl group, geranyl group, and oleyl group); cycloalkenyl groups (preferably $C_{3-30}$ substituted or unsubstituted cycloalkenyl groups, i.e., monovalent groups obtained by removing one hydrogen atom from $C_{3-30}$ cycloalkenes, such as, for example, a 2-cyclopentene-1-yl group and 2-cyclohexene-1-yl group); bicycloalkenyl groups (substituted or unsubstituted bicycloalkenyl groups, preferably $C_{5-30}$ substituted or unsubstituted bicycloalkenyl groups, i.e., monovalent groups obtained by removing one hydrogen atom from bicycloalkene groups having one double bond, such as, for example, a bicyclo[2,2,1]hept-2-ene-1-yl group and bicyclo[2,2,2]oct-2-ene-4-yl group]; alkynyl groups (preferably $C_{2-30}$ substituted or unsubstituted alkynyl groups, such as, for example, an ethynyl group, propargyl group, and trimethylsilyl ethynyl group); aryl groups (preferably $C_{6-30}$ substituted or unsubstituted aryl groups, such as, for example, a phenyl group, p-tolyl group, naphthyl group, m-chlorophenyl group, and o-hexadecanoyl aminophenyl group); heterocyclic groups (preferably substituted or unsubstituted monovalent groups having 5 or 6 members that are obtained by removing one hydrogen atom from aromatic or non-aromatic heterocyclic compounds, and more preferably $C_{3-30}$ aromatic heterocyclic groups having 5 or 6 members, such as, for example, a 2-furyl group, 2-thienyl group, 2-pyrimidinyl group, and 2-benzothiazolyl group. Further, cationic heterocyclic groups, such as a 1-methyl-2-pyridinio group and 1-methyl-2-quinolinio group may be included.); cyano groups; hydroxyl groups; nitro groups; carboxyl groups; alkoxy groups (preferably $C_{1-30}$ substituted or unsubstituted

alkoxy groups, such as, for example, a methoxy group, ethoxy group, isopropoxy group, t-butoxy group, n-octyloxy group, and 2-methoxyethoxy group); aryloxy groups (preferably $C_{6-30}$ substituted or unsubstituted aryloxy groups, such as, for example, a phenoxy group, 2-methylphenoxy group, 4-t-butylphenoxy group, 3-nitrophenoxy group, and 2-tetradecanoyl aminophenoxy group); silyloxy groups (preferably $C_{3-20}$ silyloxy groups, such as, for example, a trimethylsilyloxy group and t-butyldimethylsilyloxy group) ; heterocyclic oxy groups (preferably $C_{2-30}$ substituted or unsubstituted heterocyclic oxy group groups, such as a 1-phenyltetrazole-5-oxy group and 2-tetrahydropyranyloxy group); acyloxy groups (preferably a formyloxy group, $C_{2-30}$ substituted or unsubstituted alkylcarbonyloxy groups, and $C_{6-30}$ substituted or unsubstituted aryl carbonyloxy groups, such as, for example, a formyloxy group, acetyloxy group, pivaloyloxy group, stearoyloxy group, benzoyloxy group, and p-methoxyphenyl carbonyloxy group); carbamoyloxy groups (preferably $C_{1-30}$ substituted or unsubstituted carbamoyloxy groups, such as, for example, an N,N-dimethylcarbamoyloxy group, N,N-diethylcarbamoyloxy group, morpholino carbonyloxy group, N,N-di-n-octyl aminocarbonyl oxy group, and N-n-octyl carbamoyloxy group) ; alkoxy carbonyloxy groups (preferably $C_{2-30}$ substituted or unsubstituted alkoxy carbonyloxy groups, such as, for example, a methoxycarbonyloxy group, ethoxycarbonyloxy group, t-buthoxycarbonyloxy group, and n-octyl carbonyloxy group); aryloxy carbonyloxy groups (preferably $C_{7-30}$ substituted or unsubstituted aryloxy carbonyloxy groups, such as, for example, a phenoxy carbonyloxy group, p-methoxy phenoxy carbonyloxy group, and p-n-hexadecyloxy phenoxy carbonyloxy group); amino groups (preferably an amino group, $C_{1-30}$ substituted or unsubstituted alkylamino groups, and $C_{6-30}$ substituted or unsubstituted anilino groups, such as, for example, an amino group, methylamino group, dimethylamino group, anilino group, N-methyl-anilino group, and diphenylamino group); ammonio groups (preferably $C_{1-30}$ substituted or unsubstituted ammonio groups in which an alkyl group, aryl group, or heterocyclic group is substituted, such as, for example, a trimethylammonio group, triethylammonio group, and diphenyl methylammonio group); acylamino groups (preferably a formylamino group, $C_{1-30}$ substituted or unsubstituted alkyl carbonylamino groups, and $C_{6-30}$ substituted or unsubstituted aryl carbonylamino groups, such as, for example, a formylamino group, acetylamino group, pivaloylamino group, lauroylamino group, benzoylamino group, and 3,4,5-tri-n-octyloxy phenyl carbonylamino group); aminocarbonyl amino groups (preferably $C_{1-30}$ substituted or unsubstituted aminocarbonyl amino groups, such as, for example, a carbamoyl amino group, N,N-dimethylaminocarbonylamino group, N,N-diethylaminocarbonylamino group, and morpholinocarbonylamino group); alkoxycarbonylamino groups (preferably $C_{2-30}$ substituted or unsubstituted alkoxycarbonylamino groups, such as, for example, a methoxycarbonylamino group, ethoxycarbonylamino group, t-butoxycarbonylamino group, n-octadecyloxycarbonylamino group, and N-methyl-methoxycarbonylamino group) ; aryloxy carbonylamino groups (preferably $C_{7-30}$ substituted or unsubstituted aryloxy carbonylamino groups, such as, for example, a phenoxycarbonylamino group, p-chlorophenoxycarbonylamino group, and m-n-octyloxyphenoxycarbonylamino group); sulfamoylamino groups (preferably $C_{0-30}$ substituted or unsubstituted sulfamoylamino groups, such as, for example, a sulfamoylamino group, N,N-dimethylaminosulfonylamino group, and N-n-octylaminosulfonylamino group); alkyl and arylsulfonyl amino groups (preferably $C_{1-30}$ substituted or unsubstituted alkylsulfonyl amino groups and $C_{6-30}$ substituted or unsubstituted arylsulfonyl amino groups, such as, for example, a methylsulfonyl amino group, butylsulfonylamino group, phenylsulfonyl amino group, 2,3,5-trichlorophenylsulfonylamino group, and p-methylphenylsulfonylamino group); mercapt groups; alkylthio groups (preferably $C_{1-30}$ substituted or unsubstituted alkylthio groups, such as, for example, a methylthio group, ethylthio group, and n-hexadecylthio group); arylthio groups (preferably $C_{6-30}$ substituted or unsubstituted arylthio groups, such as, for example, a phenylthio group, p-chlorophenylthio group, and m-methoxyphenylthio group); heterocyclic thio groups (preferably $C_{2-30}$ substituted or unsubstituted heterocyclic thio groups, such as, for example, a 2-benzothiazolylthio group and 1-phenyltetrazole-5-ylthio group) ; sulfamoyl groups (preferably $C_{0-30}$ substituted or unsubstituted sulfamoyl groups, such as, for example, an N-ethylsulfamoyl group, N-(3-dodecyloxy propyl)sulfamoyl group, N,N-dimethyl sulfamoyl group, N-acetylsulfamoyl group, N-benzoylsulfamoyl group, and N-(N'-phenylcarbamoyl)sulfamoyl group); sulfo groups; alkyl and aryl sulfinyl groups (preferably $C_{1-30}$ substituted or unsubstituted alkyl sulfinyl groups and $C_{6-30}$ substituted or unsubstituted aryl sulfinyl groups, such as, for example, a methyl sulfinyl group, ethyl sulfinyl group, phenyl sulfinyl group, and p-methylphenylsulfinyl group); alkyl and aryl sulfonyl groups (preferably $C_{1-30}$ substituted or unsubstituted alkyl sulfonyl groups and $C_{6-30}$ substituted or unsubstituted aryl sulfonyl groups, such as, for example, a methyl sulfonyl group, ethyl sulfonyl group, phenyl sulfonyl group, and p-methylphenyl sulfonyl group); acyl groups (preferably a formyl group, $C_{2-30}$ substituted or unsubstituted alkyl carbonyl groups, $C_{7-30}$ substituted or unsubstituted aryl carbonyl groups, and $C_{4-30}$ substituted or unsubstituted heterocyclic carbonyl groups bonded to a carbonyl group by a carbon atom, such as, for example, an acetyl group, pivaloyl group, 2-chloroacetyl group, stearoyl group, benzoyl group, p-n-octyloxyphenylcarbonyl group, 2-pyridyl carbonyl group and 2-furylcarbonyl group); aryloxy carbonyl groups (preferably $C_{7-30}$ substituted or unsubstituted aryloxy carbonyl groups, such as, for example, a phenoxy carbonyl group, o-chlorophenoxycarbonyl group, m-nitrophenoxycarbonyl group, and p-t-butylphenoxycarbonyl group); alkoxycarbonyl groups (preferably $C_{2-30}$ substituted or unsubstituted alkoxycarbonyl groups, such as, for example, a methoxycarbonyl group, ethoxycarbonyl group, t-butoxycarbonyl group, and n-octadecyloxycarbonyl group) ; carbamoyl groups (preferably $C_{1-30}$ substituted or unsubstituted carbamoyl groups, such as, for example, a carbamoyl group, N-methylcarbamoyl group, N,N-dimethylcarbamoyl group, N,N-di-n-octylcarbamoyl group, and N-(methylsulfonyl)carbamoyl group); aryl and heterocyclic azo groups (preferably $C_{6-30}$ substituted or unsubstituted

aryl azo groups, $C_{3-30}$ substituted or unsubstituted heterocyclic azo groups, such as, for example, a phenyl azo group, p-chlorophenylazo group, and 5-ethylthio-1,3,4-thiadiazole-2-ylazo group); imido groups (preferably an N-succinimide group and N-phthalimide group); phosphino groups (preferably $C_{2-30}$ substituted or unsubstituted phosphino groups, such as, for example, a dimethylphosphino group, diphenylphosphino group, and methylphenoxyphosphino group); phosphinyl groups (preferably $C_{2-30}$ substituted or unsubstituted phosphinyl groups, such as, for example, a phosphinyl group, dioctyloxyphosphinyl group, and diethoxyphosphinyl group); phosphinyloxy groups (preferably $C_{2-30}$ substituted or unsubstituted phosphinyloxy groups, such as, for example, a diphenoxyphosphinyloxy group and dioctyloxyphosphinyloxy group) ; phosphinyl amino groups (preferably $C_{2-30}$ substituted or unsubstituted phosphinyl amino groups, such as, for example, a dimethoxyphosphinylamino group and dimethylaminophosphinylamino group); phospho groups; silyl groups (preferably $C_{3-30}$ substituted or unsubstituted silyl groups, such as, for example, a trimethylsilyl group, t-butyldimethylsilyl group, and phenyl dimethylsilyl group); hydrazino groups (preferably $C_{0-30}$ substituted or unsubstituted hydrazino grous, such as, for example, a trimethylhydrazino group); or ureide groups (preferably $C_{0-30}$ substituted or unsubstituted ureide groups, such as, for example, an N,N-dimethylureide group).

Two Ws can also cooperate to form rings (aromatic or non-aromatic hydrocarbon rings or hetero rings that can be further combined together to form a polycyclic condensed ring. Examples of the rings include, for example, a benzene ring, naphthalene ring, anthracene ring, phenanthrene ring, fluorene ring, triphenylene ring, naphthacene ring, biphenyl ring, pyrrole ring, furan ring, thiophene ring, imidazole ring, oxazole ring, thiazole ring, pyridine ring, pyrazine ring, pyrimidine ring, pyridazine ring, indolizine ring, indole ring, benzofuran ring, benzothiophene ring, isobenzofuran ring, benzimidazole ring, imidazopyridine ring, quinolizine ring, quinoline ring, phthalazine ring, naphthyridine ring, quinoxaline ring, quinoxazolin ring, isoquinoline ring, carbazole ring, phenanthridine ring, acridine ring, phenanthroline ring, thianthrene ring, chromene ring, xanthene ring, phenoxathiin ring, phenothiazine ring, and phenazine ring).

Of the aforementioned substituent groups represented by W, a substituent group having a hydrogen atom may be further substituted by the aforementioned groups after the hydrogen atom is removed. Examples of such a substituent group include $-CONHSO_2$-groups (a sulfonylcarbamoyl group and carbonylsulfamoyl group), -CONHCO-groups (a carbonylcarbamoyl group), and $-SO_2NHSO_2$-groups (a sulfonyl sulfamoyl group).

More specific examples thereof include alkylcarbonylaminosulfonyl groups (e.g., an acetylaminosulfonyl group), arylcarbonylaminosulfonyl groups (e.g., a benzoylaminosulfonyl group), alkylsulfonylaminocarbonyl groups (e.g., a methylsulfonylaminocarbonyl group), and arylsulfonylaminocarbonyl groups (e.g., a p-methylphenylsulfonylaminocarbonyl group).

As a fullerene derivative to be used preferably, a compound represented by the following general formula (2) is cited.

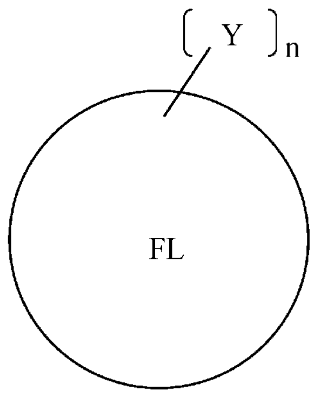

・・・(2)

In the general formula (2), FL with a circle frame represents a fullerene C60, C70, or C84. Y represents a substituent group. As the substituent group, the aforementioned W can be used. Preferred examples of the substituent group include an alkyl group, an aryl group, and a heterocyclic group, and preferred specific examples thereof include those described with respect to W. More preferred examples of the alkyl group include $C_{1-12}$ alkyl groups; and preferred examples of the aryl group and the heterocyclic group include a benzene ring, naphthalene ring, anthracene ring, phenanthrene ring, fluorene ring, triphenylene ring, naphthacene ring, biphenyl ring, pyrrole ring, furan ring, thiophene ring, imidazole ring, oxazole ring, thiazole ring, pyridine ring, pyrazine ring, pyrimidine ring, pyridazine ring, indolizine ring, indole ring, benzofuran ring, benzothiophene ring, isobenzofuran ring, benzimidazole ring, imidazopyridine ring, quinolizine ring, quinoline ring, phthalazine ring, naphthyridine ring, quinoxaline ring, quinoxazolin ring, isoquinoline ring, carbazole ring, phenanthridine ring, acridine ring, phenanthroline ring, thianthrene ring, chromene ring, xanthene ring, phenoxathiin ring, phenothiazine ring, and phenazine ring. More preferred examples thereof include a benzene ring, naphthalene ring, anthracene ring, phenanthrene ring, pyridine ring, imidazole ring, oxazole ring, and thiazole ring.

Particularly preferred examples thereof include a benzene ring, naphthalene ring, and pyridine ring. These substituent groups may further have substituent groups that may be bonded as much as possible to form rings. Herein, when n is 2 or more, multiple Ys may or may not be the same as each other, and multiple Xs may be bonded as much as possible to form rings.

n represents an integer of 1 to 60, but is preferably an integer of 1 to 10.

Hereinafter, specific examples of a fullerene derivative to be preferably used in the present embodiment will be described, but the embodiment should not be limited thereto.

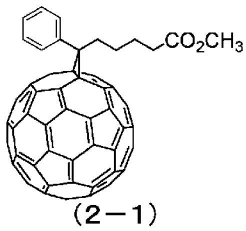
(2−1)

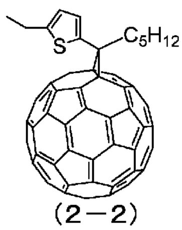
(2−2)

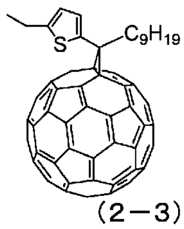
(2−3)

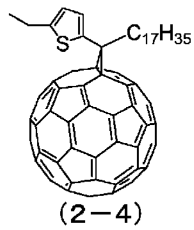
(2−4)

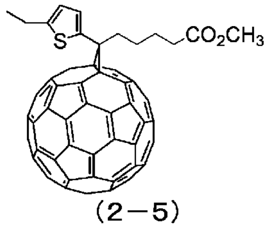
(2−5)

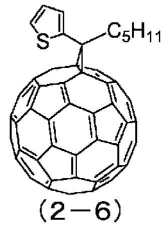
(2−6)

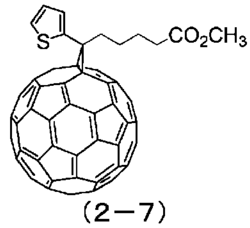
(2−7)

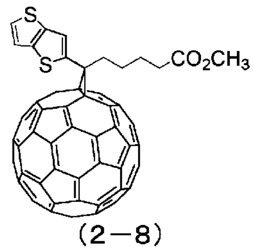
(2−8)

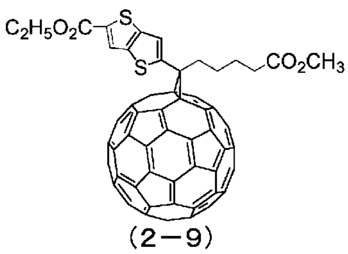
(2−9)

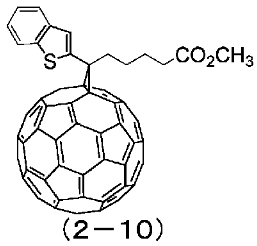
(2−10)

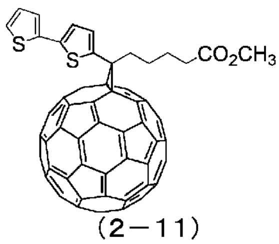
(2−11)

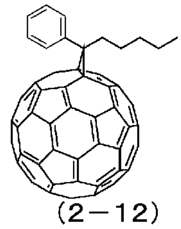
(2−12)

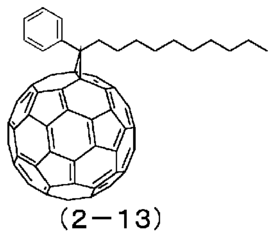
(2−13)

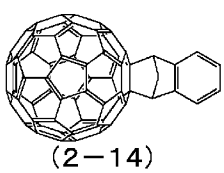
(2−14)

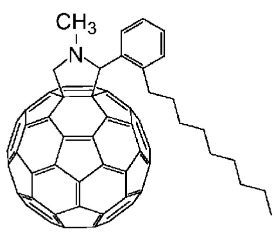
(2−15)

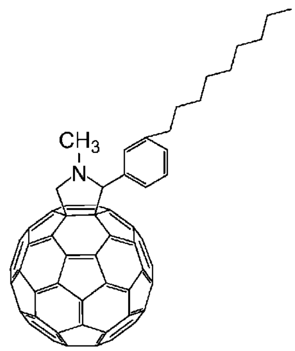
(2−16)

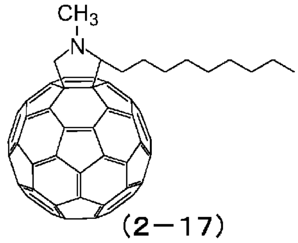
(2-17)

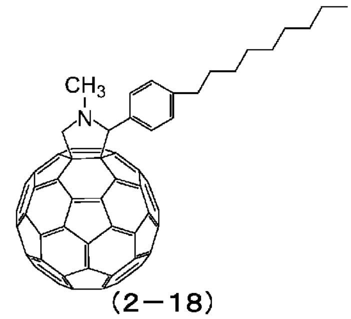
(2-18)

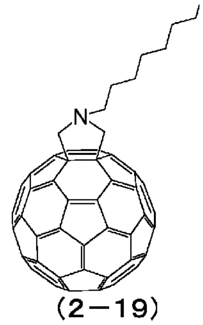
(2-19)

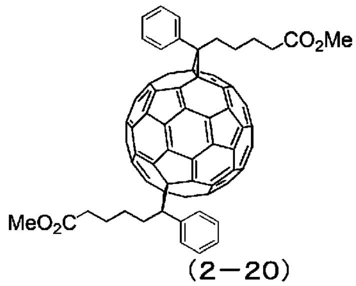
(2-20)

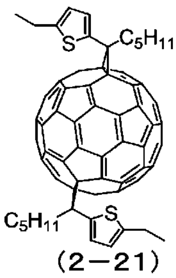
(2-21)

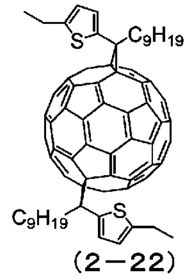
(2-22)

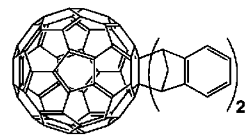
(2-23)

Among the specific examples, (2-1), (2-2), (2-3), (2-4), (2-12), (2-13), (2-20), (2-21), (2-22), and (2-23) are preferred, and (2-20), (2-21), (2-22), and (2-23) are more preferred.

Alternatively, as the fullerene and fullerene derivative to be used in the present embodiment, the compounds described in the following documents can also be used, the documents being: Quarterly Chemistry Survey, No. 43 (1999), edited by Chemical Society of Japan; Japanese Patent Application Publication No. 1998-167994; Japanese Patent Application Publication No. 1999-255508; Japanese Patent Application Publication No. 1999-255509; Japanese Patent Application Publication No. 2002-241323; and Japanese Patent Application Publication No. 2003-196881, etc. The fullerenes and fullerene compounds to be used in the embodiment can be produced, for example, by the methods described in the aforementioned documents, or by methods according to the methods described therein. Among these electron-accepting molecules, by using ICBA as the n-type organic semiconductor, the open circuit voltage ($V_{oc}$) can be improved.

When a fullerene derivative is used as the n-type organic semiconductor, it is preferable that the first reduction potential thereof is 1160 mV (vs $Fc/Fc^+$) or more, more preferable that the potential is 1250 mV (vs $Fc/Fc^+$) or more, and still more preferable that the potential is 1350 mV (vs $Fc/Fc^+$) or more. A method of measuring the first reduction potential of the n-type organic semiconductor will be described later.

The thickness of the photoelectric conversion layer 50 is not particularly limited, but it is 5 to 1000 nm, preferably 10 to 500 nm, more preferably 20 to 200 nm, and still more preferably 40 to 100 nm. There is a tendency that, as the thickness of a photoelectric conversion layer is smaller, light resistance is more improved.

The hole transport layer 60 is provided in a region between the second electrode 70 and the photoelectric conversion layer 50. The hole transport layer 60 functions to facilitate the transport of holes from the photoelectric conversion layer 50 to the second electrode 70. The hole transport layer 60 can also function such that the transport of electrons from the photoelectric conversion layer 50 to the second electrode 70 is hardly caused. The hole transport layer 60 is formed of a material having a high hole mobility, such as a charge transfer agent, a charge transfer complex, or the like. Examples of the charge transfer agent include, for example: conductive polymers, such as PEDOT (polythiophene, poly(ethylenedioxy)thiophene)/PSS (poly(styrenesulfonate)), polypyrrole, polyaniline, polyfuran, polypyridine, and polycarbazole; inorganic compounds, such as $MoO_3$ and $WO_3$; organic semiconductor molecules, such as phthalocyanine and porphyrin, and the derivatives and transition metal complexes thereof; triphenylamine compounds; and hydrazine compounds, etc. Examples of the charge transfer complex include, for example, tetra rear full Burren (TTF). The thickness of the hole transport layer 60 is not particularly limited, but it is 10 to 100 nm, and preferably 20 to 60 nm.

The second electrode 70 of the present embodiment is a positive electrode (hole extraction electrode), and is electrically connected to the photoelectric conversion layer 50 via the hole transport layer 60 on the side opposite to the light-receiving surface of the photoelectric conversion layer 50. The material of the second electrode 70 is not particularly limited, as far as the material has conductivity, but a metal, such as gold, platinum, silver, copper, aluminum, magnesium, lithium, potassium, or the like; a carbon electrode; or the like can be used. The second electrode 70 can be formed by a publicly-known method, such as a vacuum deposition method, electron beam vacuum deposition method, sputtering

method, method in which metal fine particles dispersed in a solvent are coated and the solvent is then volatilized and removed.

A means for blocking ultraviolet rays can be incorporated in the photoelectric conversion element 10. The means for blocking ultraviolet rays is not particularly limited, as far as the element can be blocked from ultraviolet rays, but examples of the means include an ultraviolet absorption layer, ultraviolet reflecting layer, and wavelength conversion layer for converting the wavelength of an ultraviolet ray into another wavelength, etc. The location, in which the means for blocking ultraviolet rays is provided, is not particularly limited, as far as the element can be blocked from ultraviolet rays, but the means is provided in one of the following ways: a layer having the aforementioned function of blocking ultraviolet rays is provided on the surface on the light emission side of the substrate; a film having the function of blocking ultraviolet rays is attached; a substrate having the function of blocking ultraviolet rays is used as a substrate on the light emission side; a layer having the function of blocking ultraviolet rays is provided between the substrate on the light emission side and the transparent conducting film; when the element has a sub-straight structure (a structure laminated from the metal electrode side), a sealing material having the function of blocking ultraviolet rays is used; and the like. A range of the wavelength of the ultraviolet ray to be blocked is not particularly limited, but it is 330 nm or less, preferably 350 nm or less, more preferably 370 nm or less, still more preferably 390 nm or less, and still more preferably 400 nm or less. For an ultraviolet ray, the transmissivity of the means is 10% or less, preferably 1% or less, and more preferably 0.1% or less.

According to the photoelectric conversion element 10 of the present embodiment, a photoelectric conversion efficiency can be improved. Further, because the electron transport layer 40 of the embodiment can be formed at a relatively low temperature ($t_1$) of $100°C \leq t_1 \leq 150°C$, the photoelectric conversion element can be formed on a plastic substrate. Furthermore, because the conductivity of the electron transport layer 40 is higher than that of a layer formed of a substance having a high insulating property, such as cesium carbonate, the electron transport layer 40 can be formed so as to have a thickness of approximately 20 to 60 nm. By forming the electron transport layer 40 so as to have a thickness of approximately 20 to 60 nm, occurrence of a short circuit can be suppressed, even if the thickness of the layer 40 is a little uneven. Thus, because an electron transport layer can be formed so as to have a large thickness, the electron transport layer 40 can be formed by using various film-forming methods, such as a spin coating method, die coating method, gravure printing method, ink-j et method, spray method, and screen printing method, thereby allowing the size of the photoelectric conversion element 10 to be made large.

Table 1 shows the conditions under which the photoelectric conversion elements of Examples 1 to 5 and Comparative Examples 1 to 10 are manufactured. Methods of manufacturing the photoelectric conversion elements of Examples 1 to 5 and Comparative Examples 1 to 10 will be described with reference to Table 1.

(Example 1)

<Formation of Negative Electrode>

An negative electrode (electron extraction electrode) was formed by cleaning a glass substrate (surface resistance value: 15 Ω/□) on which an ITO film had been formed by a sputtering method.

<Formation of Electron Transport Layer>

An electron transport layer was manufactured by a solution coating method. Specifically, zinc acetate dihydrate (made by Aldrich Co. LLC) was dissolved in 2-methoxyethanol such that the concentration thereof was 20 mg/ml, and further monoethanolamine was added (55 μl/ml) to prepare a solution. An electron transport layer was formed by spin-coating the solution on the aforementioned ITO for negative electrode at 2000 rpm (30 seconds) and then by subjecting the coated solution to a thermal treatment on a hot plate at 100°C for 5 minutes (see Table 1).

<Formation of Photoelectric Conversion Layer>

P3HT and ICBA were mixed at a mass ratio of 1.0 : 1.0, and the mixture was dissolved in o-dichlorobenzene such that the total concentration thereof was 2.5% by mass. A photoelectric conversion layer was formed by spin-coating the solution on the substrate, on which the electron transport layer had been formed, at 750 rpm (10 seconds).

<Formation of Hole Transport Layer>

$WO_3$ was vacuum-deposited on the substrate, on which the photoelectric conversion layer had been manufactured, by a resistance heating method. The thickness of the $WO_3$ layer was 10 nm. The degree of vacuum during the vacuum deposition was set to be $10^{-6}$ Torr or less.

<Formation of Positive Electrode>

Ag was vacuum-deposited on the substrate, on which up to the hole transport layer had been manufactured, by a resistance heating method. The thickness of the Ag layer was 100 nm. The degree of vacuum during the vacuum deposition was set to be $10^{-6}$ Torr or less.

<Sealing Treatment>

The photoelectric conversion element (organic solar cell element) thus manufactured was attached to cover glass by using a thermosetting sealant to obtain a sealed element.

[Table 1]

| | CONDITIONS OF MANUFACTURING PHOTOELECTRIC CONVERSION ELEMENT | | | |
|---|---|---|---|---|
| | PHOTOELECTRIC CONVERSION LAYER (ACCEPTOR MATERIAL) | ELECTRON TRANSPORT LAYER | HEAT TREATMENT TEMPERATURE [°C] | HEAT TREATMENT TIME [min] |
| COMPARATIVE EXAMPLE 1 | ICBA | $Zn(OAc)_2$(RETAINED WATER) | 25 | 5 |
| COMPARATIVE EXAMPLE 2 | ICBA | $Zn(OAc)_2$(RETAINED WATER) | 60 | 5 |
| COMPARATIVE EXAMPLE 3 | ICBA | $Zn(OAc)_2$(RETAINED WATER) | 80 | 5 |
| EXAMPLE 1 | ICBA | $Zn(OAc)_2$ | 100 | 5 |
| EXAMPLE 2 | ICBA | $Zn(OAc)_2$ | 120 | 5 |
| EXAMPLE 3 | ICBA | $Zn(OAc)_2$ | 150 | 5 |
| COMPARATIVE EXAMPLE 4 | ICBA | ZnO | 200 | 5 |
| COMPARATIVE EXAMPLE 5 | ICBA | ZnO | 300 | 5 |
| COMPARATIVE EXAMPLE 6 | ICBA | ZnO | 400 | 5 |
| COMPARATIVE EXAMPLE 7 | ICBA | $Cs_2CO_3$ | 150 | 10 |
| COMPARATIVE EXAMPLE 8 | ICBA | $Cs_2CO_3$ | 150 | 10 |
| EXAMPLE 4 | Bis-PCBM | $Zn(OAc)_2$ | 120 | 5 |
| EXAMPLE 5 | PCBM | $Zn(OAc)_2$ | 120 | 5 |
| COMPARATIVE EXAMPLE 9 | Bis-PCBM | ZnO | 300 | 5 |
| COMPARATIVE EXAMPLE 10 | PCBM | ZnO | 300 | 5 |

(Examples 2, 3)

The photoelectric conversion elements of Examples 2 and 3 were manufactured in the same way as that in Example 1, except that the heat treatment temperatures, occurring when the electron transport layers were formed, were 120°C and 150°C, respectively.

(Examples 4, 5)

The photoelectric conversion elements of Examples 4 and 5 were manufactured in the same way as that in Example 1, except that n-type organic semiconductors to be used in the photoelectric conversion layers were Bis-PCBM and PCBM, respectively.

(Comparative Examples 1 to 3)

The photoelectric conversion elements of Comparative Examples 1 to 3 were manufactured in the same way as that in Example 1, except that the heat treatment temperatures, occurring when the electron transport layers were formed, were 25°C, 60°C, and 80°C, respectively.

(Comparative Examples 4 to 6)

The photoelectric conversion elements of Comparative Examples 4 to 6 were manufactured in the same way as that in Example 1, except that the heat treatment temperatures, occurring when the electron transport layers were formed, were 200°C, 300°C, and 400°C, respectively. In each of the photoelectric conversion elements of Comparative Examples 4 to 6, the zinc acetate was changed to zinc oxide by being oxidized, because the heat treatment temperature was high.

(Comparative Example 7)

The photoelectric conversion element of Comparative Example 7 was manufactured in the same way as that in Example 1, except that the electron transport layer was formed as follows.

<Formation of Electron Transport Layer>

Cesium carbonate (made by Aldrich Co. LLC) was dissolved in 2-ethoxyethanol such that the concentration thereof was 1.86 mg/ml to prepare a solution. An electron transport layer was formed by spin-coating the solution on the aforementioned ITO for negative electrode at 5000 rpm (30 seconds) and then by subjecting the coated solution to a thermal treatment on a hot plate at 150°C for 10 minutes.

(Comparative Example 8)

The photoelectric conversion element of Comparative Example 8 was manufactured in the same way as that in Comparative Example 7, except that the concentration of cesium carbonate in the solution to be used for manufacturing the electron transport layer was ten times higher than that in Comparative Example 7. The obtained element did not exhibit a photoelectric conversion performance at all, and a yield was not able to be calculated.

(Comparative Examples 9, 10)

The photoelectric conversion elements of Comparative Examples 9 and 10 were manufactured in the same way as that in Comparative Example 5, except that n-type organic semiconductors to be used in the photoelectric conversion layers were Bis-PCBM and PCBM, respectively.

(Evaluation of Photoelectric Conversion Efficiency)

Current-voltage characteristics of the photoelectric conversion elements of Examples and Comparative Examples were measured, while simulated solar light having an illumination of 1000 W/$m^2$ was emitting at room temperature. Photoelectric conversion efficiencies of the solar cells were calculated from the obtained current-voltage characteristics. The obtained results of the photoelectric conversion efficiencies are shown in Table 2.

<Method of Evaluating Carboxyl Group Absorption Coefficient>

The carboxyl group absorption coefficient in each of the electron transport layers of Examples 1 to 5 and Comparative Examples 1 to 6, 9, and 10 were evaluated by using an FT-IR method (see Takashi Ehara*, Takafumi Otsuki, Junya Abe, Takaaki Ueno, Masahiro Ito, and Takafumi Hirayama, Phys. Status Solidi A 206, No. 9, 2139-2142 (2009)). The infrared absorption spectra of the electron transport layers were measured by using an Fourier transform

infrared spectrophotometer IRPrestige-21 made by Shimadzu Corporation. Samples, in each of which a film corresponding to each of the electron transport layers of Examples 1 to 5 and Comparative Examples 1 to 6, 9, and 10 had been formed on an ITO substrate, were used in measuring the infrared absorption spectra. The resolution of the measurement was set to be 2 $cm^{-1}$ and the accumulated number was set to be 256 times or more. In order to evaluate an absorption coefficient from the result of the infrared absorption spectrum measurement, the thickness of the electron transport layer was also evaluated. The thickness thereof was measured by using a contact-type film thickness meter (Dektak, etc.) or an electron microscope.

An average absorbance A between wave numbers 1200 $cm^{-1}$ and 2000 $cm^{-1}$ of the obtained infrared absorption spectrum was calculated. The straight line, passing through the two points at the wave numbers of 1200 $cm^{-1}$ and 2000 $cm^{-1}$, was used as the base line of integration. The carboxyl group absorption coefficient a in an electron transport layer was calculated from the determined average absorbance A and the thickness according to the following equation. The obtained results of the carboxyl group absorption coefficients and the thicknesses are shown in Table 2.

$$a = -\frac{1}{l}\ln(1 - A)$$

Herein, when the carboxyl group absorption coefficient in the electron transport layer incorporated in the photoelectric conversion element is evaluated, the electron transport layer is made to be present on an electrode formed of a transparent conducting film by peeing off one side of the glass or film located in the outermost layer of the photoelectric conversion element and then by dissolving and removing the sealing material and the photoelectric conversion layer with a solvent. The carboxyl group absorption coefficient can be determined by performing FT-IR measurement on the electron transport layer surface with the use of the aforementioned method.

(Measurement of Open Circuit Voltage)

Voc (open circuit voltage) of each of the photoelectric conversion elements of Examples and Comparative Examples was measured. The obtained results of the open circuit voltages are shown in Table 2.

(Yield)

Yields of the photoelectric conversion elements of Examples and Comparative Examples were calculated as follows, the obtained results of which are shown in Table 2:

(1) Ten or more of the same elements were manufactured to evaluate a photoelectric conversion efficiency;
(2) after an element, the photoelectric conversion efficiency of which is clearly low due to a short circuit, etc., is removed, the average of the photoelectric conversion efficiencies is calculated; and
(3) assuming that a photoelectric conversion element, the photoelectric conversion efficiency of which is 75% or more of the calculated average, is a good product, an yield is calculated from the equation: yield [%] = 100 × the number of good products/the total number.

(Measurement of Ionization Potential of Electron Transport Layer)

The ionization potential of the electron transport layer was measured by using an atmosphere photoelectron spectrometer (Model AC-3 made by Riken Keiki Co., Ltd.). Specifically, the ionization potential was measured by coating an electron transport layer solution on a glass substrate and then by subjecting the coated solution to a heat treatment. Herein, when the ionization potential of the electron transport layer incorporated in the photoelectric conversion element is evaluated, the electron transport layer is made to be present on an electrode formed of a transparent conducting film by peeing off one side of the glass or film located in the outermost layer of the photoelectric conversion element and then by dissolving and removing the sealing material and the photoelectric conversion layer with a solvent. The ionization potential is measured from the electron transport layer surface by the aforementioned method.

(Identification of Oxidation-Reduction Potential of N-type Material of Photoelectric Conversion Layer)

With reference to "Electochemical Methods: Fundamentals and Applications" (edited by A. J. Bard), the procedures for identifying the oxidation-reduction potential of the n-type material of the photoelectric conversion layer were performed as follows. A o-dichlorobenzene 0.1M solution of tetrabutylammonium perchlorate was manufactured, and 4 mg of ferrocene was added, as an internal reference substance, to per 50 mL of the solution to prepare a measuring solution. To 2 mL of this solution, 1.5 mg of a fullerene derivative was added, and an oxidation-reduction potential was

measured at a sweep rate of 20 mV/s by using a potentiostat galvanostat (electrochemical analyzer: model 630A made by ALS). The first reduction potential was determined as the average of the first reduction peak and its oxidation peak, based on the oxidation/reduction potential ($Fc/Fc^+$) of the ferrocene added as an internal reference substance. Herein, the oxidation-reduction potential of the n-type material of the photoelectric conversion layer incorporated in the photoelectric conversion element was identified as follows : after one side of the cover glass in the photoelectric conversion element was peeled off and the photoelectric conversion layer was dissolved with o-dichlorobenzene, tetrabutylammonium perchlorate and 4 mg of ferrocene, as an internal reference substance, were added to per 50 mL of the solvent to prepare a measuring solution. With the use of the obtained solution, an oxidation-reduction potential was measured at a sweep rate of 20 mV/s by using a potentiostat galvanostat (electrochemical analyzer: model 630A made by ALS). The first reduction potential was determined as the average of the first reduction peak and its oxidation peak, based on the oxidation/reduction potential ($Fc/Fc^+$) of the ferrocene added as an internal reference substance.

[Table 2]

| | EVALUATION RESULTS | | | | | | |
|---|---|---|---|---|---|---|---|
| | CARBOXYL GROUP ABSORPTION COEFFICIENT [$cm^{-1}$] | PHOTOELECTRIC CONVERSION EFFICIENCY[%] | OPEN CIRCUIT VOLTAGE [V] | FILM THICKNESS [nm] | YIELD [%] | IONIZATION POTENTIAL [eV] | OXIDATION-REDUCTION POTENTIAL OF n-TYPE MATERIAL OF PHOTOELECTRIC CONVERSION LAYER [mV] |
| COMPARATIVE EXAMPLE 1 | 4.7E+05 | 0.01 | 0.217 | 30 | 0 | 5.5 | 1350 |
| COMPARATIVE EXAMPLE 2 | 3.6E+05 | 0.001 | 0.262 | 30 | 0 | 5.7 | 1350 |
| COMPARATIVE EXAMPLE 3 | 3.0E+05 | 0.08 | 0.441 | 30 | 0 | 5.8 | 1350 |
| EXAMPLE 1 | 2.3E+05 | 5.06 | 0.803 | 30 | >95 | 5.9 | 1350 |
| EXAMPLE 2 | 1.7E+05 | 5.03 | 0.799 | 30 | >95 | 5.9 | 1350 |
| EXAMPLE 3 | 7.5E+04 | 5.01 | 0.793 | 30 | >95 | 6.0 | 1350 |
| COMPARATIVE EXAMPLE 4 | 3.4E+04 | 3.66 | 0.689 | 30 | >95 | 6.1 | 1350 |
| COMPARATIVE EXAMPLE 5 | 2.4E+04 | 3.17 | 0.611 | 30 | >95 | 6.2 | 1350 |
| COMPARATIVE EXAMPLE 6 | 0.0E+00 | 0.002 | 0.020 | 30 | 0 | 6.2 | 1350 |
| COMPARATIVE EXAMPLE 7 | - | 4.41 | 0.733 | <10 | 65 | 2.0 | 1350 |
| COMPARATIVE EXAMPLE 8 | - | 0 | 0 | 30 | 0 | 2.0 | 1350 |
| EXAMPLE 4 | 1.7E+05 | 3.80 | 0.680 | 30 | >95 | 5.9 | 1250 |
| EXAMPLE 5 | 1.7E+05 | 3.49 | 0.576 | 30 | >95 | 5.9 | 1160 |
| COMPARATIVE EXAMPLE 9 | 2.4E+04 | 2.75 | 0.580 | 30 | >95 | 6.2 | 1250 |

(continued)

| | EVALUATION RESULTS | | | | | | |
|---|---|---|---|---|---|---|---|
| | CARBOXYL GROUP ABSORPTION COEFFICIENT [$cm^{-1}$] | PHOTOELECTRIC CONVERSION EFFICIENCY[%] | OPEN CIRCUIT VOLTAGE [V] | FILM THICKNESS [nm] | YIELD [%] | IONIZATION POTENTIAL [eV] | OXIDATION-REDUCTION POTENTIAL OF n-TYPE MATERIAL OF PHOTOELECTRIC CONVERSION LAYER [mV] |
| COMPARATIVE EXAMPLE 10 | 2.4E+04 | 3.00 | 0.545 | 30 | >95 | 6.2 | 1160 |
| (※) IN EACH OF THE ELEMENTS OF COMPARATIVE EXAMPLES 1 TO 3, 6, AND 8, PHOTOELECTRIC CONVERSION WAS NOT EXHIBITED AT ALL OR WAS EXTREMELY LOW. AND NO GOOD PRODUCT WAS OBTAINED, AND HENCE YIELDS WERE 0%. | | | | | | | |

As shown in Table 2, it has been confirmed that the photoelectric conversion efficiency of the photoelectric conversion element of each Example is remarkably improved and the open circuit voltage thereof is also remarkably increased by using zinc acetate in the electron transport layer and by combining it with ICBA having a low LUMO level, in comparison with the photoelectric conversion element of each Comparative Example.

In the photoelectric conversion element of each Example, the solution for forming the electron transport layer can be easily prepared, and the layer can be easily formed by coating the solution to the substrate. Accordingly, the time for manufacturing the photoelectric conversion element can be shortened and mass production of the elements can be achieved.

In the photoelectric conversion element of each Example, the conductivity of the electron transport layer is sufficient for transporting electrons, in comparison with that of $Cs_2CO_3$, and hence the electron transport layer can be formed so as to have a large thickness (30 nm in Examples). Accordingly, occurrence of a short circuit between the photoelectric conversion layer and the negative electrode can be suppressed, leading to an increase in yield. Further, by forming the electron transport layer so as to have a large thickness, occurrence of a short circuit can be suppressed, even if the thickness of the layer is a little uneven. Because the electron transport layer can be formed so as to have a large thickness, it can be formed, in addition to the aforementioned spin coating method, by using various film-forming methods, such as a die coating method, thereby allowing the size of the photoelectric conversion element to be made large.

In the photoelectric conversion element of each Example, the heat treatment temperature, at which the electron transport layer is formed, is a relatively low temperature range of 100°C to 150°C, and hence a plastic substrate having low heat resistance, and in particular, a flexible substrate can be used as the substrate. As a result, the weight of the photoelectric conversion element can be reduced. Further, by using an inexpensive plastic substrate, the manufacturing cost of the photoelectric conversion element can be reduced. Furthermore, by using a flexible substrate as the substrate, the plasticity and flexibility of the photoelectric conversion element can be further enhanced. As a result, the applications of the photoelectric conversion element can be extended.

The present invention should not be limited to the aforementioned embodiments, and various modifications, such as design modifications, can be made with respect to the embodiments based on the knowledge of those skilled in the art, and an embodiment with such a modification can be encompassed within the scope of the present invention.

In the photoelectric conversion element 10 according to each embodiment, although the electron transport layer 40 is provided between the photoelectric conversion layer 50 and the first electrode 30 and the hole transport layer 60 is provided between the photoelectric conversion layer 50 and the second electrode 70, for example, the location of the hole transport layer 60 and that of the electron transport layer 40 can be replaced with each other. When the electron transport layer 40 is provided in a region between the second electrode 70 and the photoelectric conversion layer 50 and the hole transport layer 60 is provided in a region between the first electrode 30 and the photoelectric conversion layer 50, the first electrode 30 serves as a positive electrode and the second electrode 70 as a negative electrode.

Reference Numerals

10 PHOTOELECTRIC CONVERSION ELEMENT
20 SUBSTRATE
30 FIRST ELECTRODE
40 ELECTRON TRANSPORT LAYER
50 PHOTOELECTRIC CONVERSION LAYER
60 HOLE TRANSPORT LAYER
70 SECOND ELECTRODE

Industrial Applicability

The present invention can be used in the fields related to a photoelectric conversion element configured to convert light energy into electrical energy by photoelectric conversion.

## Claims

**1.** A photoelectric conversion element comprising:

a photoelectric conversion layer;
an electron extraction electrode provided on one major surface side of the photoelectric conversion layer;

a hole extraction electrode provided on the other major surface side of the photoelectric conversion layer; and an electron transport layer provided between the photoelectric conversion layer and the electron extraction electrode, wherein
the electron transport layer contains a substance represented by the following chemical formula and a reactant thereof:

M(X)a (1)

wherein M is selected from the group consisting of alkali metals, alkaline earth metals, group 2B and 3B metals, and transition metals; X is selected from a halogen, a carboxylate group, an alkoxy group, an alkyl group, and an acetonate group represented by the following formula; and a is a positive integer determined in accordance with the valence of M:

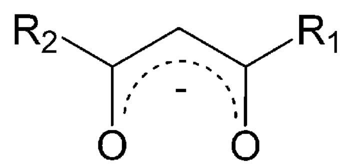

wherein $R_1$ and $R_2$ are selected from hydrogen, a $C_{1-20}$ linear or branched alkyl group, and a $C_{1-20}$ linear or branched alkoxy group, and $R_1$ and $R_2$ may or may not be the same as each other.

2. The photoelectric conversion element according to claim 1, wherein
X in the chemical formula (1) is a carboxylate group or an acetonate group, and a carboxyl group absorption coefficient ($\alpha_1$) in the electron transport layer is $0.5 \times 10^5 cm^{-1} \le \alpha_1 \le 2.5 \times 10^5 cm^{-1}$.

3. The photoelectric conversion element according to claim 1 or claim 2, wherein
an ionization potential of the electron transport layer is 6.2 eV or less.

4. The photoelectric conversion element according to any one of claims 1 to 3, wherein
the electron transport layer contains one or more metal compounds and a reactant thereof, the metal compounds being selected from the group consisting of zinc acetate, magnesium acetate, aluminum acetylacetonate, aluminum chloride, gallium acetylacetonate, gallium chloride, zinc acetylacetonate, zinc chloride, and diethylzinc.

5. The photoelectric conversion element according to claim 1, wherein
the photoelectric conversion layer includes a fullerene derivative having a first reduction potential of 1160 mV (vs $Fc/Fc^+$) or more.

6. The photoelectric conversion element according to claim 5, wherein
the fullerene derivative is ICBA (bisindenyl C60).

7. A method of manufacturing a photoelectric conversion element including a pair of electrodes, a photoelectric conversion layer located between the pair of electrodes, and an electron transport layer provided between one of the electrodes and the photoelectric conversion layer, the method comprising:

forming the electron transport layer by heating a film at a temperature ($t_1$) of $100°C \le t_1 \le 150°C$ after the film is formed by coating a solution containing a substance represented by the following chemical formula:

M(X)a (1)

wherein M is selected from the group consisting of alkali metals, alkaline earth metals, group 2B and 3B metals, and transition metals; X is selected from a halogen, a carboxylate group, an alkoxy group, an alkyl group, and an acetonate group represented by the following formula; and a is a positive integer determined in accordance with the valence of M:

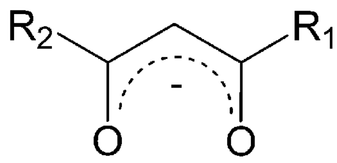

wherein $R_1$ and $R_2$ are selected from hydrogen, a $C_{1-20}$ linear or branched alkyl group, and a $C_{1-20}$ linear or branched alkoxy group, and $R_1$ and $R_2$ may or may not be the same as each other.

FIG. 1

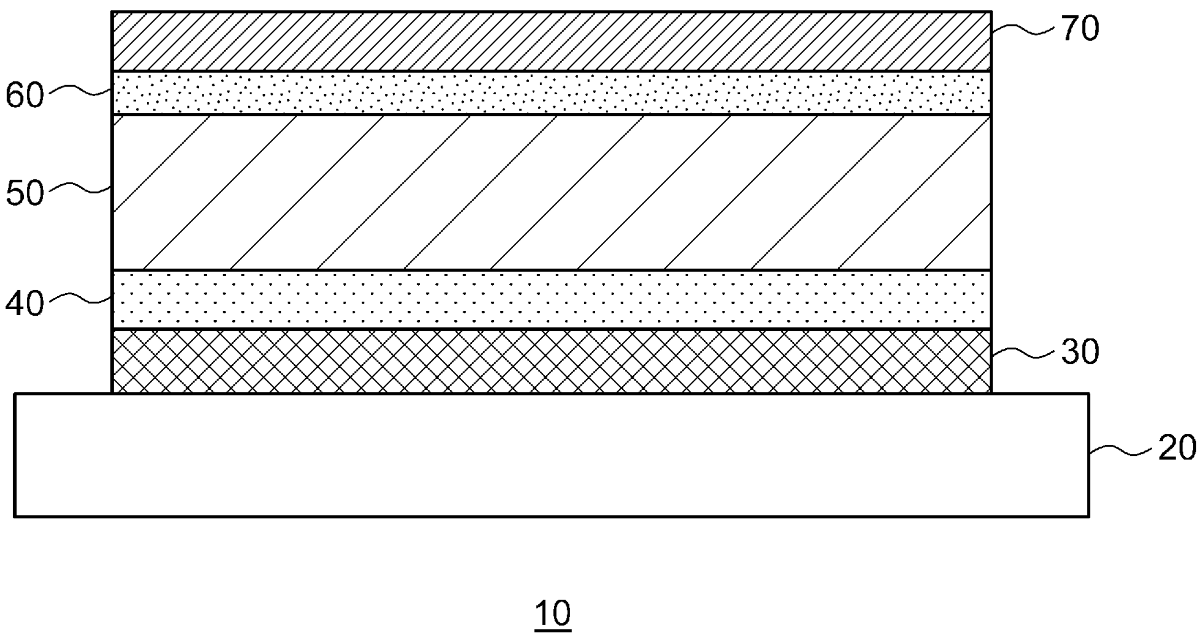

**INTERNATIONAL SEARCH REPORT**

International application No.
PCT/JP2012/005147

A. CLASSIFICATION OF SUBJECT MATTER
*H01L51/42*(2006.01)i, *C07C49/92*(2006.01)i

According to International Patent Classification (IPC) or to both national classification and IPC

B. FIELDS SEARCHED

Minimum documentation searched (classification system followed by classification symbols)
H01L31/04-31/078, 51/42, C07C49/92

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)
IEEE Xplore, CiNii, JSTPlus(JDreamII), JST7580(JDreamII)

C. DOCUMENTS CONSIDERED TO BE RELEVANT

| Category* | Citation of document, with indication, where appropriate, of the relevant passages | Relevant to claim No. |
|---|---|---|
| Y | JP 2011-35386 A (Sumitomo Chemical Co., Ltd.), 17 February 2011 (17.02.2011), claim 1; paragraphs [0002], [0088], [0100], [0109], [0117], [0157], [0178] (Family: none) | 1-7 |
| Y | Combination of Indene-C60 Bis-Adduct and Cross-Linked Fullerene Interlayer Leading to Highly Efficient Inverted Polymer Solar Cells, J.Am.Chem.Soc., 2010, Vol.132, No.49, PP.17381-17383 | 1-7 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>15 October, 2012 (15.10.12) | Date of mailing of the international search report<br>23 October, 2012 (23.10.12) |
| Name and mailing address of the ISA/<br>Japanese Patent Office<br>Facsimile No. | Authorized officer<br><br>Telephone No. |

Form PCT/ISA/210 (second sheet) (July 2009)

**INTERNATIONAL SEARCH REPORT**

International application No.
PCT/JP2012/005147

C (Continuation). DOCUMENTS CONSIDERED TO BE RELEVANT

| Category* | Citation of document, with indication, where appropriate, of the relevant passages | Relevant to claim No. |
|---|---|---|
| A | JP 2011-124468 A (Konica Minolta Holdings, Inc.), 23 June 2011 (23.06.2011), paragraphs [0024], [0028], [0053], [0061], [0126], [0133], [0142], [0145], [0149]; fig. 2 (Family: none) | 1-7 |
| A | JP 2003-332075 A (President of Tokyo Institute of Technology), 21 November 2003 (21.11.2003), claim 10; paragraphs [0052], [0088], [0096] (Family: none) | 1-7 |
| A | Characterization of ZnS-layer-inserted bulk-heterojunction organic solar cells by ac impedance spectroscopy, Journal of Applied Physics, 2009, Vol.105, PP.124513-1-124513-6 | 1-7 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 10167994 A
- JP 11255508 A
- JP 11255509 A
- JP 2002241323 A
- JP 2003196881 A

### Non-patent literature cited in the description


- **A. K. K .KYAW ; X. W. SUN ; C. Y. JIANG ; G. Q. LO ; D. W. ZHAO ; D. L. KWONG.** *Applied Physics Letters,* 2008, vol. 93, 221107
- **P. DE BRUYN ; D. J. D. MOET ; P. W. M. BLOM.** *Organic Electronics,* 2010, vol. 11, 1419-1422
- **C. WALDAUF ; M. MORANA ; P. DENK ; P. SCHILINSKY ; K. COAKLEY ; S. A. CHOULIS ; C. J. BRABEC.** *Applied Physics Letters,* 2006, vol. 89, 233517
- **HUA-HSIEN LIAO ; LI-MIN CHEN ; ZHENG XU ; GANG LI ; YANG YANG.** *APPLIED PHYSICS LETTERS,* 2008, vol. 92, 173303
- Quarterly Chemistry Survey. 1999
- **TAKASHI EHARA ; TAKAFUMI OTSUKI ; JUNYA ABE ; TAKAAKI UENO ; MASAHIRO ITO ; TAKAFUMI HIRAYAMA.** *Phys. Status Solidi A,* 2009, vol. 206 (9), 2139-2142